# EUROPÄISCHE PATENTSCHRIFT

(11) **EP 1 176 971 B1**
(45) Veröffentlichungstag und Bekanntmachung des Hinweises auf die Patenterteilung: **08.09.2004**
(21) Anmeldenummer: 00927102.4
(22) Anmeldetag: 28.04.2000
(51) Int. Cl.: A61K 35/78, A61P 1/16

(54) **VERWENDUNG VON PHYLLANTHUS ZUR BEHANDLUNG VON CHRONISCH ENTZÜNDLICHEN UND FIBROTISCHEN PROZESSEN**
USE OF PHYLLANTHUS FOR TREATING CHRONIC INFLAMMATORY AND FIBROTIC PROCESSES
UTILISATION DE PHYLLANTHUS DANS LE TRAITEMENT DE PROCESSUS A CARACTERE FIBREUX ET INFLAMMATOIRE CHRONIQUE

(30) Priorität: 29.04.1999 DE 19919585
(43) Veröffentlichungstag der Anmeldung: 06.02.2002
(73) Patentinhaber: Phytrix AG, 80335 München (DE)
(72) Erfinder: WAGNER, Hildebert, D-83254 Breitbrunn (DE); VOLLMAR, Angelika, D-81375 München (DE); MANNS, Michael, D-30916 Isernhagen (DE); GEBHARDT, Rolf, D-04103 Leipzig (DE); BAHR, Matthias, D-31303 Burgdorf (DE); BUNIATIAN, Gayane, Hrachia, D-04103 Leipzig (DE)
(74) Vertreter: VOSSIUS & PARTNER
(86) Internationale Anmeldenummer: PCT/EP2000/003869
(87) Internationale Veröffentlichungsnummer: WO 2000/066134

(56) Entgegenhaltungen:
- DATABASE BIOSIS [Online] BIOSCIENCES INFORMATION SERVICE, PHILADELPHIA, PA, US; 1997 ZHOU SHIWEN XU CHUANFU ET AL: "Mechanism of protective action of Phyllanthus urinaria L. against injuries of liver cells." Database accession no. PREV199799511060 XP002144251 & ZHONGGUO ZHONGYAO ZAZHI, Bd. 22, Nr. 2, 1997, Seiten 109-111, 129, ISSN: 1001-5302
- DATABASE BIOSIS [Online] BIOSCIENCES INFORMATION SERVICE, PHILADELPHIA, PA, US; 1998 ASHA V V ET AL: "Preliminary evaluation of the antihepatotoxic activity of Phyllanthus kozhikodianus, P. maderaspatensis and Solanum indicum." Database accession no. PREV199800438007 XP002144252 & FITOTERAPIA, Bd. 69, Nr. 3, 1998, Seiten 255-259, ISSN: 0367-326X
- DATABASE BIOSIS [Online] BIOSCIENCES INFORMATION SERVICE, PHILADELPHIA, PA, US; 6. Februar 1999 (1999-02-06) JEENA K JOSE ET AL: "Effect of Emblica officinalis, Phyllanthus amarus and Picrorrhiza kurroa on N-nitrosodiethylamine induced hepatocarcinogenesis." Database accession no. PREV199900216982 XP002144253 & CANCER LETTERS, Bd. 136, Nr. 1, 6. Februar 1999 (1999-02-06), Seiten 11-16, ISSN: 0304-3835

## Beschreibung

Die vorliegende Erfindung betrifft die Verwendung von Phyllanthus zur Herstellung eines Arzneimittels zur Prävention oder Behandlung von Bindegewebsvermehrungen. Die Beschreibung der vorliegenden Erfindung stellt weiterhin das, wie Phyllanthus zur Erhaltung des Spiegels an reduziertem Glutathion, zur Inhibierung der Lipopolysaccharid (LPS)-induzierten Stickstoffmonoxid-Synthase (NOS) sowie zur Inhibierung der Expression des Cyclooxygenase (COX-2) Proteins verwendet werden kann.

Phyllanthus umfaßt eine weitverbreitete Gruppe von Pflanzen, die in Zentral- und Südindien, Taiwan, sowie Bereichen Zentral- und Mittelamerikas beheimatet ist. Unter dem Begriff Phyllanthus im Sinne dieser Erfindung sind dabei alle Vertreter der botanischen Familie Phyllanthus zu verstehen, wie Phyllanthus niruri oder insbesondere Phyllanthus amarus etc. Aus der Volksmedizin Indiens ist es bekannt, eine Vielzahl von Krankheiten mit Phyllanthus zu behandeln. So zählt beispielsweise in Band I "Doktor K.M. Nadkarni's Indian Materia Medica (3^{rd} edition; revised and enlarged by A.K. Nadkarni)" der Autor auf, daß die Pflanze als de-obstruent, diuretisch, adstringent und kühlend bekannt ist. Ebenso werden Zusammensetzungen mit Phyllanthus zur Behandlung von Ikterus, Wassersucht, Tripper, Menorrhagie und anderen den Urogenitaltrakt betreffenden Beeinträchtigungen ähnlicher Art beschrieben. Bekannt sind weiter die Verwendung des Saftes des Stengels gemischt mit Öl als Ophthalmica oder Anwendungen gegen Geschwüre, zur Behandlung von Wunden und Schwellungen etc., wie auch die Verwendung der Blätter zur Behandlung von Juckreiz oder anderen Hautbeeinträchtigungen.

Weiter ist eine Vielzahl von Wirksubstanzen bekannt, die aus Phyllanthus isoliert werden können, Phyllanthin, Hypophyllanthin, Triacontanol, Triacontanal, Repandusinsäure A (s. beispielsweise JP 03206044 A; AIDS-Res-Hum-Retroviruses (11/1992), Bd. 8 (11), Phyllantostatin-1, Phyllantosid, Phyllantocin, Phyllantocinsäure (s. beispielsweise EP- 173 4480; US 4,388,457), Phyllamycin A, B und C, Retrojusticidin B, Justicidin A und B (s. beispielsweise AIDS-Weekly, 25.9.95, AIDS Therapies Extracts), Linolsäure, Linolensäure und Ricinoleinsäure (s, beispielsweise Journal-of-the-American-Oil-Chemists-Society Ausg. 81.06.00, Riconoleic acid in Phyllanthus niruri), Phyllamyricin D, E und F, Phyllamyricosid A, B und C (s. beispielsweise J-Nat-Prod. (11/1996), Bd. 59 (11), Six lignans from Phyllanthus niruri), Putranjivain A (s. beispielsweise Chem-Pharm-Bull (Tokyo), (04/1995), Bd. 43 (4), Inhibitory effects), Ursulinsäure und Nirorisid (s. beispielsweise J-Nat-Prod 02/96, Bd. 59 (2), Niruriside; Recl. Trav. Chim. (06/1996).

An therapeutischen Wirkungen und Anwendungen sind bisher bekannt eine altersverzögemde Wirkung (s. beispielsweise JP 08176004), Vorbeugung und Therapie von Immunschwächen wie AIDS oder Krankheiten wie Influenza, Erkältungen, TBC, Hepatitis, Zirrhosen (s. beispielsweise US 5,529,778; AIDS-Weekly-Plus v. 05.08.96, Antiviral (Drug Development); Inhibition of HIV), antineoplastische Wirkung (s. beispielsweise US 4,388,457), Therapie von HIV-, HBVund/oder HCV-Infektionen, insbesondere topische Behandlung des Karposi Sarkoms (s, beispielsweise EP 1734480; US 5,466,455), Wirkung als Proteaseinhibitor, Elastaseinhibitor und als Bleichmittel (s. beispielsweise JP 09087136), analgetische und entzündungshemmende Wirkung, Wirkung als Tyrosinase-Inhibitor (s. beispielsweise JP 08012566) und eine Verwendung als Desinfektionsmittel in Kombination mit Extrakten aus anderen Pflanzen. Im übrigen sind auch Verwendungen in kosmetischen Zubereitungen bekannt. An dieser Vielzahl von verschiedenen Anwendungsgebieten und isolierten Wirkstoffen ist zu erkennen, daß Phyllanthus eine durchaus bekannte Gattung von Heilpflanzen ist, die für eine Vielzahl von Indikationen und Beschwerden eingesetzt wird.

Ein pathologisches Phänomen, das für eine Vielzahl von anderen Beschwerden verantwortlich zu sein scheint, ist der sogenannte oxidative Streß. Darunter versteht man die Belastung der lebenden Zelle durch Anreicherung giftiger oxidierter Verbindungen, so wie Lipidhydroperoxide, Wasserstoffperoxid, singulären Sauerstoff und Hydroxyl-Hyperoxid-Anionen. Dabei kann der Streß durch lokal produzierte oder von außen zugeführte Radikale, insbesondere sog. Reaktive Sauerstoff-Species (ROS) oder Peroxonitrit-Radikale etc. hervorgerufen werden. Der oxidative Streß kann z.B. auch durch Strahlungseinwirkung, Xenobiotika, Schwermetallionen oder Ischämie-Reperfusion (zeitweise Unterbrechung der Blutzufuhr eines Organs) induziert werden. In letzterem Falle werden durch die Xanthinoxidase, einer zu den Flavoproteinen gehörenden Oxidase von ca. 300 kD, die den Abbau der Purine katalysiert, im reichen Maße Hyperoxid-Anionen gebildet, da ihr natürlicher Elektronenakzeptor Sauerstoff ist. Unter physiologischen Bedingungen werden diese Hyperoxid-Anionen durch Superoxid-Dismutase deaktiviert, bei einer Reperfusion allerdings entstehen dabei nachgewiesenermaßen große Mengen an Sauerstoffradikalen.

Oxidativer Streß spielt eine wichtige Rolle bei der Entstehung einer Reihe akuter und insbesondere chronischer Erkrankungen, z.B. Entzündungen verschiedener Art, Mikroangiopathien, Fibrose, rheumatoide Arthritis und andere rheumatische Erkrankungen, Arteriosklerose (LDL-Oxidation), Tumorentstehung und -progression, möglicherweise der Alzheimerschen Krankheit, aber auch arzneimittelinduzierte akute Schäden, wie die Paracetamol-Schädigung der Leber.

Dabei spielt die Leber als zentrales dynamisches Organ des Körpers eine wichtige Rolle in einer großen Zahl der genannten physiologischen und mikrophysiologischen Prozesse, wobei die Stoffwechselaktivitäten der Leber (Intermediärstoffwechsel) von entscheidender Bedeutung einerseits für die Versorgung weiterer Organe, andererseits aber auch für die chemische Umsetzung (Verstoffwechselung) von pharmazeutisch aktiven Substanzen sind (s. Pschyrembel, Klinisches Wörterbuch, de Gruyter Verlag, 1986, S. 935 - 937). Das bereits beschriebene Phänomen des oxidativen Streß wird ebenfalls größtenteils vom Körper in der Leber bekämpft. Hier befindet sich ein Reservoir unterschiedlicher reduzierter Verbindungen von Antioxidantien, z.B. L-Ascorbinsäure, Karotinoide, Dihydroliponsäure, Harnsäure, Glutathion oder α-Tocopherol, welche mit Hilfe verschiedener Enzymaktivitäten (z.B. Superoxid-Dismutase, Peroxidasen wie Glutathionperoxidase, Katalase etc.) das Auftreten reaktiver Radikale verhindern.

Dabei wirkt sich oxidativer Streß besonders nachteilig auf eine Vielzahl von Funktionen des Lebergewebes aus. Das Lebergewebe reagiert darauf häufig mit Bindegewebsvennehrungen, welche die weitere Progression einer nachhaltigen Leberschädigung, wie beispielsweise die Entwicklung eines Lebertumors begünstigt. Dabei sind bei der Pathogenese der hepatotoxischen Wirkung vor allem Gallensäuren beteiligt.

Bei all den oben genannten Erkrankungen kommt der Balance zwischen oxidativem Streß und den Abwehrsystemen der Zellen und Organe entscheidende Bedeutung zu. Es ist daher von entscheidender prophylaktischer wie therapeutischer Bedeutung, einerseits die gesunde Leber vor oxidativem Streß zu schützen, andererseits eine erkrankte Leber zu stärken, so daß sie bereits bestehenden oxidativen Streß nachhaltig überwinden kann.

Verbindungen mit leberschutzender Wirksamkeit haben z.T. erhebliche Nachteile, weil sie bei einer bereits erkrankten Leber aufgrund einer zu hohen Toxizität nicht angewendet werden können.

Der vorliegenden Erfindung liegt daher die Aufgabe zugrunde, leberaktive Substanzen bereitzustellen, die sowohl eine prophylaktische als auch eine therapeutische Wirkung aufweisen.

Ein Gegenstand der vorliegenden Erfindung ist die Verwendung von Phyllanthus zur Herstellung eines Arzneimittels zur Prävention oder Behandlung von Bindegewebsvermehrungen, insbesondere von fibrotischen Veränderungen z.B. der Leber, der Lunge, der Niere, des Pankreas, des Darms, von endokrinen Organen, der Milz, des männlichen oder weiblichen Urogenitaltraktes, der Gelenke z.B. als Folge chronisch entzündlicher Prozesse, wie z.B. der rheumatoiden Arthritis oder chronischer Kardiomyopathien, sowie von Zirrhosen, einem fortgeschrittenen Stadium von Fibrosen.

Besonders bevorzugt ist die Verwendung gemäß der vorliegenden Erfindung daher bei Fibrosen und Zirrhosen, vorzugsweise Leberfibrose und Leberzirrhose. Dabei führen insbesondere chronische Entzündungszustände zu Gewebeschwund und ausgeprägter Narbenbildung mit fortschreitendem Funktionsverlust der Organe. Eine Hemmung oder Prävention der Bindegewebsvermehrung führt daher zu einer weniger ausgeprägten Narbenbildung und einem Erhalt der Funktionsfähigkeit der Organe.

Dabei geht die entsprechende Wirksamkeit von Phyllanthus bei der Verhinderung bzw. Verbesserung gerade der Leberfibrose vermutlich auf eine antioxidative Wirkung zurück, wobei die Ursache von Leberfibrosen häufig in viralen Infektionen liegen. So besitzen beispielsweise alle bekannten Hepatitisviren (Hepatitis A, B, C, D, E und wahrscheinlich auch G) einen ausgesprochenen Tropismus für Leberzellen. Es ist davon auszugehen, daß selbst die derzeit in der Medizin verwendeten antiviralen Medikamente nicht zu einer Viruselimination sondern lediglich zur Unterdrückung der Virusvermehrung (Virussuppression) führen. Selbst beim Verschwinden des Virus im peripheren Blut (unterhalb der Nachweisgrenze) ist das Virus oftmals noch im Lebergewebe nachweisbar. Daher kann Phyllanthus hier durch seine prophylaktische als auch therapeutische Wirkung einen vorteilhaften Effekt auf die Leberregeneration ausüben. Dabei wird zur Verminderung von chronisch entzündlichen Prozessen beigetragen, wobei die sich entwickelnde Bindegewebsvermehrung in der Leber herabgesetzt wird. Bisher sind keine Medikamente bekannt, die bereits in einem so frühen Schritt einer degenerativen Entwicklung eingreifen können.

Die Beschreibung der vorliegenden Erfindung erläutert die Verwendung von Phyllanthus zur Erhaltung des Spiegels an reduziertem Glutathion. Es konnte überraschenderweise eine starke Wirksamkeit von Phyllanthusextrakten in der Aufrechterhaltung des Spiegels an reduziertem Glutathion, welches vor allem in der Leber vorkommt, gezeigt werden. Im Rahmen dieser Versuche wurde festgestellt, daß ein Extrakt von Phyllanthus in den Funktionszellen der Leber (Hepatozyten), die eine durch t-Butyl-Hydroperoxid gesteigerte Lipidperoxidation aufwiesen, nicht nur die weitere Lipidperoxidation unterdrückt, sondern sogar die endogene Lipidperoxidation fast vollständig aufgehoben wird. In vergleichenden Versuchen mit unbehandelten Hepatozyten wurde eine klare Zunahme der reduktiven Kapazität festgestellt, was auf eine verbesserte Aufrechterhaltung des intrazellulären Spiegels an reduziertem Glutathion schließen läßt.

Die Beschreibung der vorliegenden Erfindung erläutert, wie Phyllanthus verwendet, wird um die Expression von Smooth-Muscle-alpha-Aktin (SMA) mRNA und SMA-Protein zu verringern. In einer fibrotischen Leber, die eine erhöhte Zellteilungsrate aufweist, kommt es zur Anreicherung (Akkumulation) von extrazellulärer Matrix. Die erhöhten Mengen an extrazellulärer Matrix werden als entscheidend für die weitere Progression einer Leberfibrose, bis hin zur Leberzirrhose erachtet. Die Anreicherung von extrazellulärer Matrix geht zurück auf die Aktivierung von speziellen Leberzellen, den hepatische Sternzellen (HSC), die in aktivierter Form als aktivierte HSC bezeichnet werden. Aktivierte HSC produzieren im Vergleich zu nicht aktivierten HSC größere Mengen an Smooth-Muscle-alpha-Aktin (SMA) mRNA und Protein, weshalb sich die Aktivierung von HSC an der Expression von SMA messen läßt. Außerdem läßt sich der Aktivierungszustand von HSC aus der Expression und intrazellulären Verteilung des Glial Fibrillary Acidic Protein (GFAP) ableiten. In Versuchsreihen konnte nun überraschenderweise festgestellt werden, daß HSC nach Behandlung mit Phyllanthusextrakte zu deutlich geringeren Mengen an extrahierbarer SMA-mRNA und SMA-Protein führen. Außerdem läßt sich mittels Immunfluoreszenz an Hand der Verteilung von SMA und GFAP nachweisen, daß Phyllanthusextrakte den normalen Phänotyp der HSC stabilisieren und die Vergrößerung der Zellen im aktivierten Zustand verhindern. Ferner zeigten mit Phyllanthusextrakten behandelte HSC eine deutliche Inhibition des Zellwachstums, was die Wirksamkeit von Phyllanthusextrakten in diesen Experimenten unterstreicht. Damit besteht eine Möglichkeit aktivierte HSC, wie sie in fibrotischer Leber vorkommen, wieder in nicht aktivierte HSC zu überführen, um damit eine Regression der Leberfibrose zu begünstigen.

Die Beschreibung der vorliegenden Erfindung erläutert die Verwendung von Phyllanthus zur Inhibierung der Lipopolysaccharid (LPS)-induzierten Stickstoffmonoxid-Synthase (NOS), zur Inhibierung der induzierten Stickstoffinonoxid-Synthase (iNOS) sowie die Verwendung von Phyllanthus zur Inhibierung der Expression des Cyclooxygenase (COX-2) Proteins.

LPS ist eine Sammelbezeichnung für Konjugate, die aus Lipid- und Polysaccharidanteilen zusammengesetzt sind. Die in der äußeren Membran der Zellwände Gram-negativer Bakterien vorkommenden LPS sind prinzipiell aus drei Komponenten aufgebaut, nämlich Lipid A, dem Kern-Oligosaccharid und den O-spezifischen Seitenketten. Das Lipid A verankert das LPS in der bakteriellen Zellwand und ist ferner für die immunaktivierende Wirkung von bakteriellen Zellwandbestandteilen verantwortlich.

Mit Hilfe von LPS kann in Leberzellen die Expression von iNOS und COX-2 induziert werden, weshalb die stimulierten Zellen als Modellsysteme für fibrotische Leberzellen verwendet werden können, in denen die Expression dieser beiden Proteine ebenfalls erhöht ist. Sowohl iNOS als auch COX-2 ist als potenter Mediator von entzündlichen Prozessen, wie sie bei degenerativen Veränderungen der Leber auftreten, bekannt. In vergleichenden Experimenten konnte nun überraschenderweise gefunden werden, daß Leberzellen, die mit LPS stimuliert und anschließend mit Phyllanthusextrakten behandelt wurden, eine deutliche Reduktion der Expressionsraten des iNOS- und des COX-2 Proteins zeigten.

Ein weiterer Gegenstand dieser Erfindung umfaßt die Verwendung in einer der oben genannten Weisen, wobei eine aus Phyllanthus isolierte Fraktion verwendet wird. Unter einer isolierten Fraktion versteht man in diesem Sinne eine beispielsweise durch chromatographische Mittel, Destillation, Präzipitation, Extraktion, Filtration oder in sonstiger Weise aus Phyllanthus abgetrennte Untermenge von Phyllanthus-Substanzen. Darunter insbesondere zu verstehen sind Extrakte sowie deren durch Chromatographie, Destillation, Präzipitation bzw. Extraktion abgetrennte Fraktionen.

Eine weitere erfindungsgemäße Verwendung umfaßt Verwendungen gemäß einem der vorgenannten Beispiele, bei dem eine oder mehrere aus Phyllanthus isolierte chemische Substanzen, insbesondere Wirkstoffe, verwendet werden. Darunter sind insbesondere auch aus Phyllanthus-Extrakten oder sonstigen Auszügen isolierte Einzelsubstanzen, sogenannte Naturstoffisolate, zu verstehen, wie sie beispielsweise auch aus dem Stand der Technik bekannt sind. Die Verwendung dieser isolierten Wirkstoffe birgt den Vorteil, daß im allgemeinen mit beträchtlich geringeren Substanzmengen gearbeitet werden muß und dabei oft spezifischere Wirkungen als mit Gesamtextrakten oder Tabletten erreicht werden.

Bei bevorzugten Verwendungen nach einer der erfindungsgemäßen Verwendungsformen ist Phyllanthus ausgewählt aus einzelnen Mitgliedern der Familie Phyllanthus, aus der Gruppe Phyllanthus amarus, Phyllanthus niruri, Phyllanthus emblica, Phyllanthus urinaria, Phyllanthus myrtifolius Moon, Phyllanthus maderas pratensis und/oder Phyllanthus ussuriensis.

In den erfindungsgemäßen Verwendungen können Blätter, Rinde, Blüten, Samen, Früchte, Stengel, Äste, Stamm, Wurzel und/oder Holz von Phyllanthus verwendet werden, vorzugsweise die Herba-Droge, d.h. alle oberirdischen Teile der Pflanze. Dabei kann die Verwendung von Phyllanthus in zerkleinerter Form und/oder in unveränderter Form, d.h. als ganzes Blatt, als Granulat, Pulver, Präzipitat, Extrakt, getrockneter Extrakt und /oder Exsudat erfolgen, wobei Extrakte oder getrocknete Extrakte bevorzugt sind.

Die Herstellung von Phyllanthus zur erfindungsgemäßen Verwendung umfaßt die Herstellung von Phyllanthuspulver oder -granulat aus einem der vorgenannten Pflanzenteile, Extraktion aus Pflanzen, zerkleinerten Pflanzenteilen, Pulvern, sowie auch an bereits vorher mit anderen Lösungsmitteln behandelten Resten mit Hexan, Wasser, Methanol und/oder anderen Alkoholen. Dazu gehören auch Filtration und Vakuum-Evaporation, um einen getrockneten Extrakt zu erhalten. Eine weitere Methode umfaßt die mehrphasige Extraktion mit wäßrigen und/oder alkoholischen und/oder polaren Lösungsmitteln. Üblich ist auch die Filtration, beispielsweise durch Zellulosefilter, die Präzipitation, vorzugsweise mit Hilfe von Ethanol, oder die Trennung durch Ultrazentrifugation sowie eine Mazeration. Dabei kann stets bei erhöhten oder erniedrigten Temperaturen gearbeitet werden.

Besonders bevorzugt ist hier die Verwendung von Phyllanthus in Form eines wäßrigen, lipophilen oder alkoholischen Extrakts, wobei der alkoholische Extrakt, vorzugsweise mit kurzkettigen (C1 bis C4) primären Alkoholen oder Mischungen davon, insbesondere Methanol oder Ethanol, durchgeführt wird, der lipophil mit C5-C10, verzweigt oder unverzweigt, kettigen Kohlenwasserstoffen, oder Mischungen davon, vor allem mit n-Hexan.

Desweiteren eignen sich als Extraktionsmittel Essigsäureethylester oder entsprechende organische Lösungsmittel/Wasser-Mischungen, vorzugsweise Methanol/Wassermischungen oder Ethanol-/Wassermischungen. Ein geeignetes Extraktionsverfahren ist beispielsweise im U.S. Patent No. 4,673,575 oder U.S. Patent No. 4,937,074 offenbart.

Phyllanthus wird bei erfindungsgemäßen Verwendungen bevorzugt in Form eines oder mehrerer Heilmittel (s. Römp, Lexikon Chemie, Version 1.4), wie einer Infusionslösung, Injektionslösung, Tablette, eines Granulats, einer Salbe, einer Heilpackung, Klysmen und/oder in Form eines oder mehrerer Lebensmittel/Nahrungsergänzungsmittel eingesetzt. Damit umfaßt sind die üblichen medizinischen und therapeutischen Anwendungen und insbesondere auch die als Nahrungsergänzungsmittel, wobei hier zur Prophylaxe und auch als funktionelles Antioxidans Phyllanthus als natürlicher und ungefährlicher Zusatz zu Nahrungsmitteln verwendet werden kann und dabei auch präventiv die genannten therapeutischen und funktionellen Effekte zeigt.

Die Verwendung von Phyllanthus in den erfindungsgemäßen Verwendungen kann oral, topisch, und/oder parenteral erfolgen.

In einer bevorzugten Ausführungsform wird zusätzlich zu Phyllanthus noch ein oder mehrere andere Wirkstoffe und/oder geeignete Zusatz- und/oder Hilfsstoffe verwendet. Unter dem Begriff Wirkstoff werden im Sinne dieser Erfindung therapeutisch wirksame Stoffe wie z.B. Vitamin C oder Tocopherole, insbesondere α-Tocopherol, verstanden, die als Antioxidantien oder als Wirkstoffe gegen oxidativen Streß bekannt sind, sowie z.B. entzündungshemmende Stoffe. Damit umfaßt sind damit auch sog. Kombipräparate mit Phyllanthus. Dabei ist insbesondere auch zu beachten, daß die erfindungsgemäßen Verwendungen keineswegs nur auf jeweils eine Form, Fraktion oder isolierten Wirkstoff von Phyllanthus beschränkt sind, bei einer Verwendung auch mehrere verschiedene Formen und/oder Fraktionen und/oder isolierten Wirkstoff von Phyllanthus verwendet werden können.

Unter Hilfs- und Zusatzstoffen versteht man im Sinne dieser Erfindung Stoffe, die bekanntermaßen für therapeutische Anwendungen oder als Anwendung zur Nahrungsergänzungsmittel hinzugefügt werden, um einen entsprechenden Einsatz zu erlauben oder erleichtern, so z.B. Adjuvantien, Spreng- und Gleitmittel, Füllstoffe, Puffer, Konservierungsmittel, Stabilisatoren etc..

Die folgenden Beispiele und Figuren sollen die Erfindung näher beschreiben, ohne sie zu beschränken. Darin zeigt:
- Figur 1: die Ergebnisse von Radikalfänger-Versuchen mit 1,1-Diphenyl-2-picrylhydrazyl (DPPH) und den erfindungsgemäßen Phyllanthusextrakten;
- Figur 2: die Ergebnisse von Radikalfänger-Versuchen mit DPPH und Vitamin C als Vergleich;
- Figur 3: die Ergebnisse von Radikalfänger-Versuchen mit DPPH und α-Tocopherol als Vergleich;
- Figur 4: die Ergebnisse von Radikalfänger-Versuchen mit 3-[4,5-Dimethylthiazol-2-yl]-2,5-diphenyl-tetrazolium-bromid (MTT) und den erfindungsgemäßen Phyllanthusextrakten;
- Figur 5: die Ergebnisse von Radikalfänger-Versuchen mit MTT und Vitamin C als Vergleich;
- Figur 6: die Ergebnisse von Radikalfänger-Versuchen mit Cytochrom und den erfindungsgemäßen Phyllanthusextrakten;
- Figur 7: die Ergebnisse von Radikalfänger-Versuchen mit Cytochrom und Vitamin C als Vergleich;
- Figur 8: die Ergebnisse von Radikalfänger-Versuchen mit Natrium-3'[I-[(phenylamino)-carbonyl]-3,4-tetrazolium]-bis(4-methoxy-6-nitro)-benzen-sulfonsäurehydrat/Phenanzin methosulfat (XTT/PMS) und den erfindungsgemäßen Phyllanthusextrakten;
- Figur 9: die Ergebnisse von Radikalfänger-Versuchen mit XTT/PMS und Vitamin C als Vergleich;
- Figur 10a-c: die Wirkung von Phyllanthusextrakten auf Leberzellen (Hepatozyten);
- Referenzfigur 11: die inhibierende Wirkung von Phyllanthusextrakten auf die LPS-induzierte Expression der NOS anhand der reduzierten NO Produktion;
- Referenzfigur 12: die inhibierende Wirkung eines Ethanol/Wasser-Extraktes von Phyllanthus auf die LPS-induzierte iNOS Expression;
- Referenzfigur 13: die inhibierende Wirkung eines Hexan-Extraktes von Phyllanthus auf die LPS-induzierte COX-2 Expression; und
- Referenzfigur 14: die inhibierende Wirkung eines Ethanol/Wasser-Extraktes von Phyllanthus auf die LPS-induzierte COX-2 Expression;

### Beispiel 1:

### Herstellung eines erfindungsgemäßen getrockneten Extraktes

### (Fraktion 1; Lot-No.: 9810H)

2 kg der Herba-Droge eines in Madras, Indien kultivierten Phyllanthus amarus wurde zu 450 g Pulver verarbeitet. Diese 450 g Pulver wurden für 12 h mit 3 l destilliertem n-Hexan in einem Soxhlet Apparat extrahiert. Nach Filtration und Vakuumevaporation wurden 25 g getrockneter n-Hexan-Extrakt gewonnen, dessen Hauptbestandteile lipophile Lignane, Sterole und Pigmente waren. Der Extrakt war eine grau-braune Paste, die in Wasser und Methanol unlöslich und in Ethyl-Acetat löslich war.

### Beispiel 2:

### Herstellung eines getrockneten Methanol-Extraktes

### (Fraktion 2; Lot-No.: 9810M)

Der Rest des n-Hexan unlöslichen Pflanzenrückstands aus Beispiel 1 wurde 24 h sukzessive mit 3 l destilliertem Methanol in einem Soxhlet-Apparat extrahiert. Nach Filtration und Vakuumevaporation wurden 50 g getrockneter Methanol-Extrakt gewonnen. Die Hauptbestandteile sind Flavonoide, oligomere Gallotannine und Phenolcarbonsäuren. Das dunkelbraune Pulver war unlöslich in Wasser und fast komplett in Methanol löslich.

### Beispiel 3:

### Herstellung eines wässrigen Überstandes

### (Fraktion 3; Lot-No.: 9810Ws)

Der nach Methanolextraktion (s. Beispiel 2) zurückbleibende methanolunlösliche Pflanzenrest (ca. 375 g) wurde mit 2,5 l destilliertem Wasser heiß infundiert und dann über 12 h kalt mazeriert (+4° C). Nach Filtration des warmen Wassserextraktes wurden 2,5 l Ethanols (100% (=1:1)) tröpfchenweise hinzugefügt, um ein Präzipitat der hochmolekularen Disaccharide und Glykoproteine zu erreichen, die durch Ultrazentrifugation bei 7500 rpm getrennt wurden. Die lyophilisierte überstehende Phase ergab 15 g Trockenmaterial. Hauptbestandteile waren oligomere Gallotannine und andere wasserlösliche Polymere. Ergebnis war ein rot-braunes Pulver, das in Wasser löslich und unlöslich in organischen Lösungsmittel war.

### Beispiel 4:

### Herstellung eines Wasserpräzipitats

### (Fraktion 4; Lot-No.: 9810pp)

Die Ethanol-präzipitierten und zentrifugierten polymeren Anteile gem. Beispiel 3 wurden in heißem Wasser gelöst und lyophilisiert. Ausgehend von 450 g rohem Pulvers werden 5 g Extrakt gewonnen. Hauptbestandteile sind hochmolekulare Polysaccharide und Glykoproteine. Das entstehende Produkt war ein braunes Pulver, daß in Wasser unlöslich war.

### Beispiel 5:

### Herstellung eines chlorophyllfreien Methanol-Rohextraktes

### (Fraktion 5; Lot-No.: 9901Mcf; P1159)

100 g Phyllanthuspulver aus der Herba-Droge wurden mit 500 ml destilliertem Methanol in einem Soxhlet-Apparat für 12 h extrahiert. Filtration und Vakuumevaporation lieferte 15 g getrockneten Extrakt, der in 300 ml einer Mischung aus destilliertem Wasser und Methanol im Verhältnis 1:1 heiß aufgelöst wurde. Die Lösung wurde auf einem Wasserbad von 80°C unter gelegentlichem Umrühren auf das halbe Volumen reduziert. Das ölige Präzipitat wurde durch Heißfiltration (80°C) durch Zellulosepapier entfernt. Zum Filtrat wurde heißes destilliertes Wasser (80°C) zugegeben und bis 300 ml aufgefüllt und danach erneut filtriert. Lyophilisation des Filtrats ergab ca. 10g getrockneten Methanolextrakt. Dieser hatte das Aussehen einer braunen Paste und war in Methanol löslich.

### Beispiel 6:

### Herstellung eines chlorophyllfreien Wasserextraktes

### (Fraktion 6; Lot-No.: 9901 Wcf)

50 g Phyllanthus-Pulver aus der Herba-Droge wurden 1 h mit 500 ml destilliertem Wasser auf einem Wasserbad von 80-100° C unter gelegentlichem Rühren ausgekocht. Die heiße Lösung wurde durch ein Zellulosepapier gefiltert. Zu dem Filtrat (ca. 400 ml) wurden 100 ml destilliertes Methanol hinzugefügt. Die Mixtur wurde unter gelegentlichem Rühren auf einem Wasserbad von 80°C auf das halbe Volumen evaporiert. Das ölige Präzipitat wurde durch heiße Filtration (80°C) durch Zellulosepapier abgetrennt. Lyophilisieren des Filtrats ergab 3g getrockneten Wasserextraktes. Dieser Extrakt war ein rot-braunes Pulver, das in Methanol löslich war.

### Beispiel 7:

### Radikalfängerversuch mit DPPH

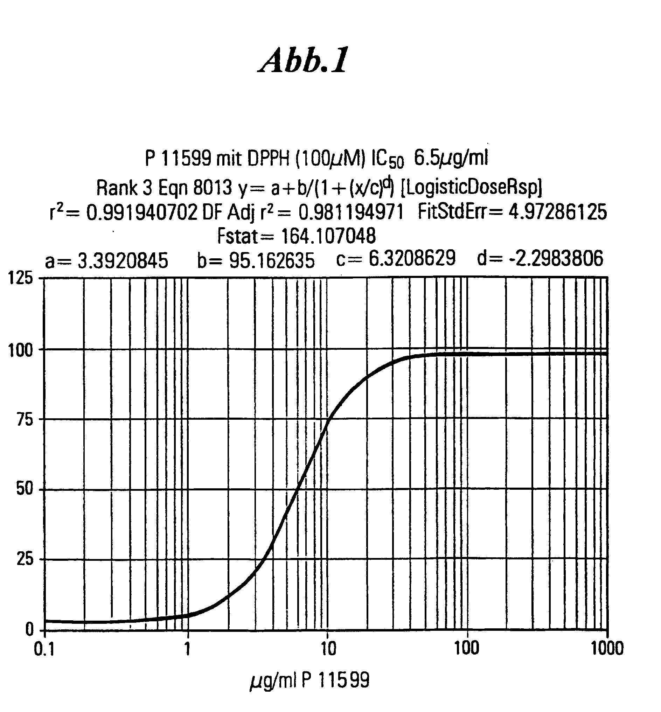

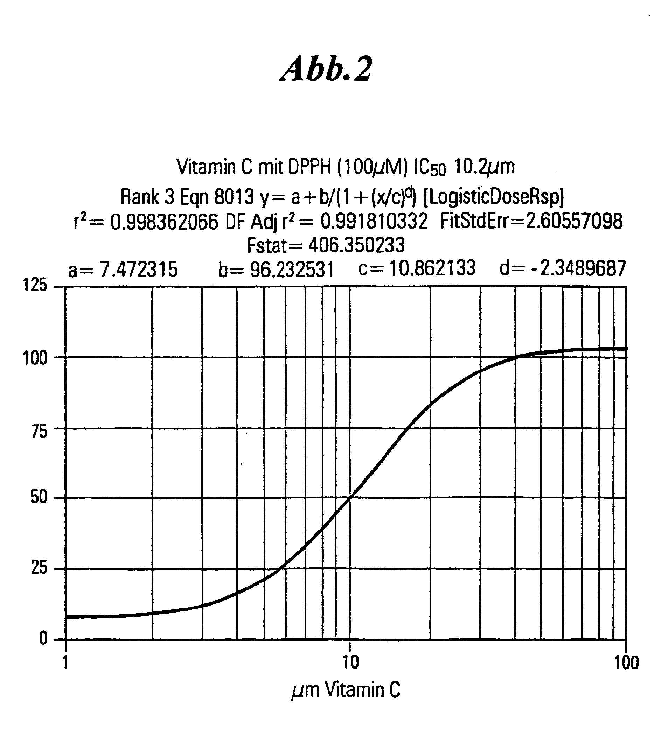

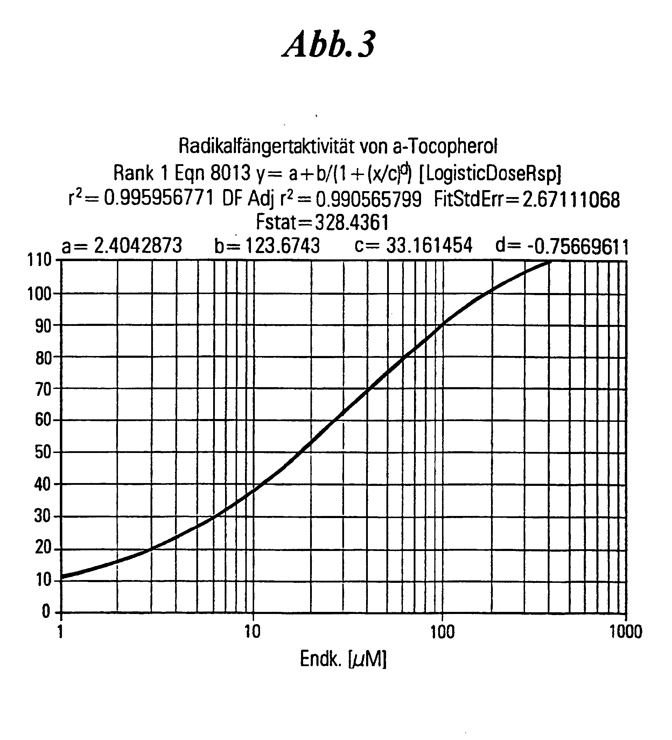

7 verschiedene Konzentrationen eines erfindungsgemäßen Extraktes genannt P 11599 gem. Beispiel 5 wurden in einem Radikalfängerversuch auf ihre Fähigkeit zum Abfangen von Radikalen untersucht. Dazu wurde der Farbumschlag zwischen einem stabilen Radikal DPPH (1,1-Diphenyl-2-picrylhydrazyl) und dem zugehörigen Nicht-Radikal 1,1-Diphenyl-2-picrylhydrazin,bei 515 nm gemessen. Dabei wurden die Testsubstanzen in DMSO in Verdünnungsreihen und Doppelbestimmungen mit einer DPPH-Lösung in Methanol über 30 min bei 37°C inkubiert und der Farbumschlag gemessen. Aus den Ergebnissen wurde der SC50-Wert bestimmt, die Konzentration an Probe, bei der 50% der DPPH-Radikale abgefangen werden. DMSO wurde als negative und Ascorbinsäure als positive Kontrolle verwendet und - wie auch α-Tocopherol - vermessen. Als SC50-Werte wurden 6,2 µg/ml für P11599, 10 µM für Ascorbinsäure, und 18 µM für a-Tocopherol (s. Abb. 1 - 3) bestimmt. Wie man den Kurven und Ergebnissen entnehmen kann, war P11599 ein besserer Radikalfänger als α-Tocopherol und der Ascorbinsäure ebenbürtig.

### Beispiel 8:

### Radikalfängerversuch mit MTT

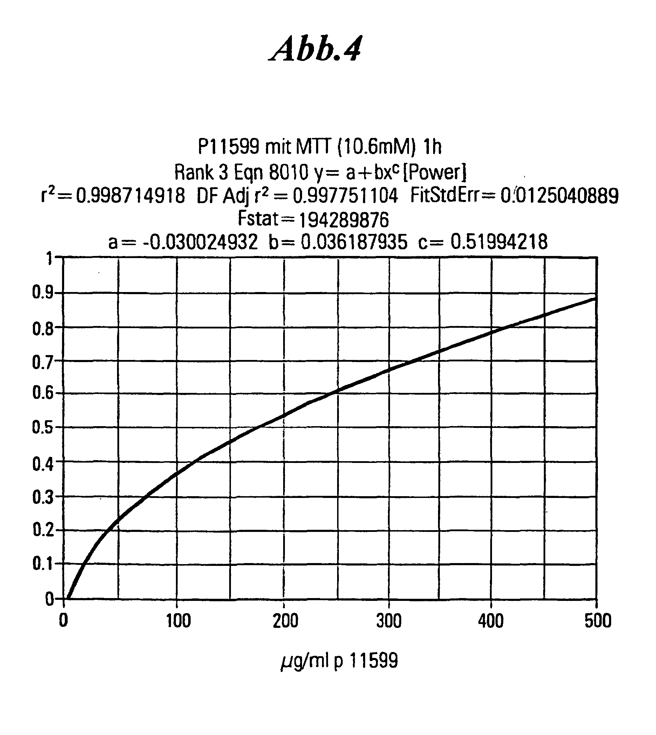

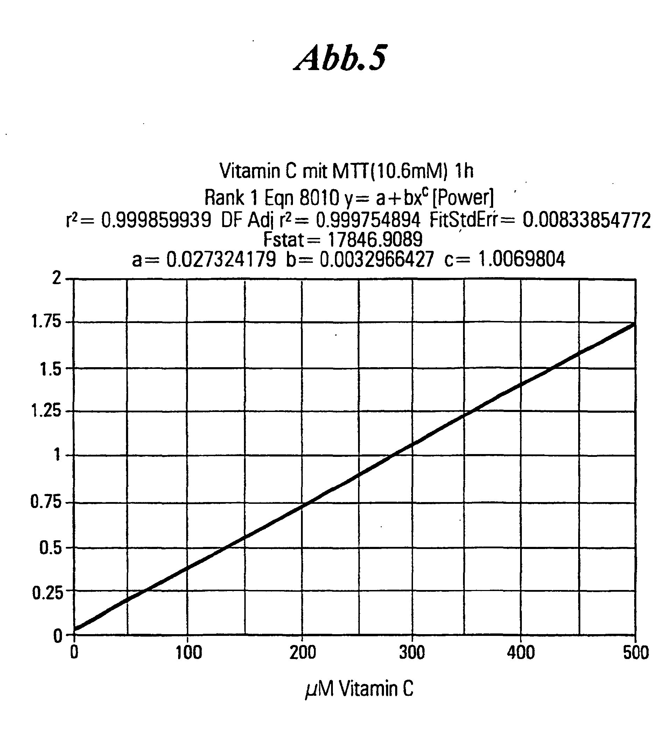

MTT (3-[4,5-Dimethyl-thiazol-2-yl]2,5-diphenyl-tetrazolium-bromid) wurde in einer Konzentration von 10,6 mM 1 h bei 37°C mit verschiedenen Konzentrationen eines erfindungsgemäßen Extrakts P11599 gem. Beispiel 5 bzw. mit Ascorbinsäure als Referenz inkubiert und die Änderung der Absorption durch die Wirkung der Proben photometrisch bestimmt. Als Ergebnis ist eine außerordentlich hohes antioxidatives Potential von P11599 festzustellen, das dem des relativ starken Reduktionsmittels Ascorbinsäure ähnelt (s. Abb. 4 und 5).

### Beispiel 9:

### Radikalfängerversuch mit Cytochrom c

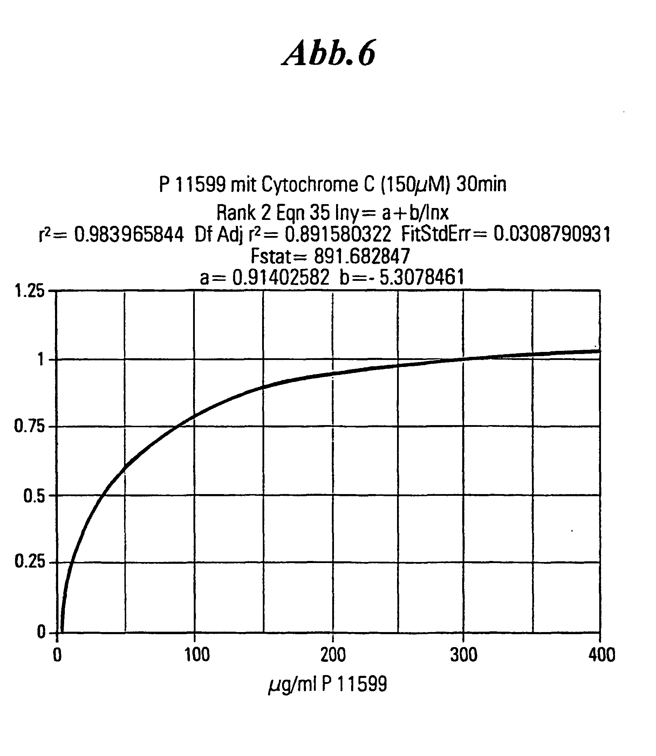

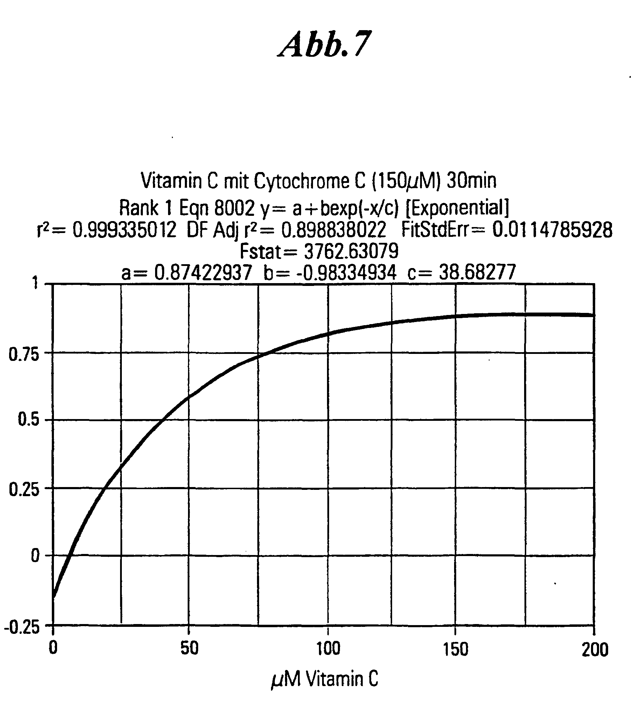

Cytochrom c wurde in einer Konzentration von 150 uM 0,5 h bei 37°C mit verschiedenen Konzentrationen eines erfindungsgemäßen Extrakts P11599 gem. Beispiel 5 bzw. mit Ascorbinsäure als Referenz inkubiert und die Änderung der Absorption durch die Wirkung der Proben photometrisch bestimmt. Als Ergebnis ist eine außerordentlich hohes antioxidatives Potential von P11599 festzustellen, das dem des relativ starken Reduktionsmittels Ascorbinsäure ähnelt (s. Abb. 6 und 7).

### Beispiel 10:

### Radikalfängerversuch mit XTT/PMS

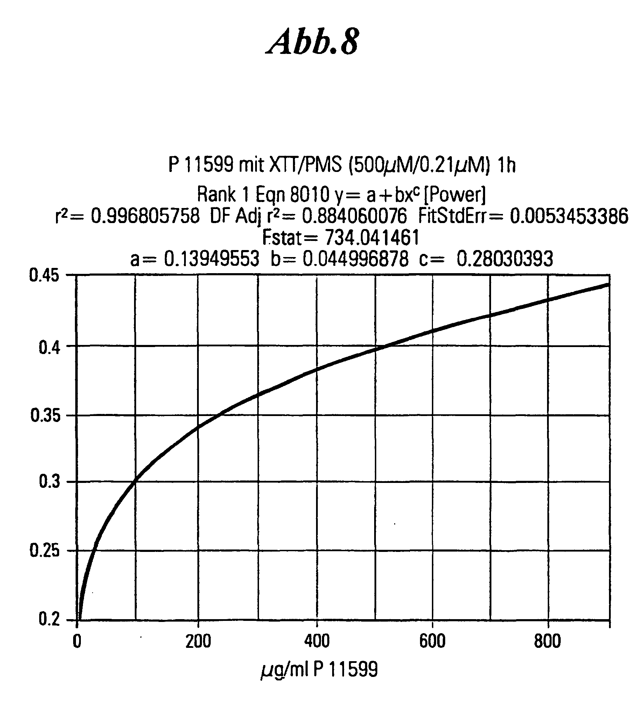

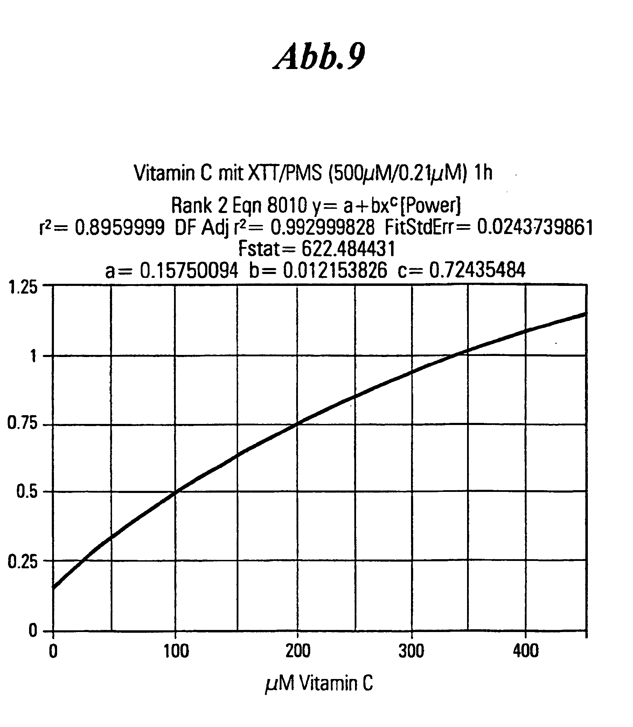

XTT/PMS (Natrium 3'[I-[(phenylamino)-carbonyl]-3,4-tetrazolium]-bis(4-methoxy-6-nitro)benzen-sulfonsäurehydrat/Phenanzin methosulfat) wurde in einer Konzentration von 500 µM/0,2 1) 1 h bei 37°C mit verschiedenen Konzentrationen eines erfindungsgemäßen Extrakts P11599 gem. Beispiel 5 bzw. mit Ascorbinsäure als Referenz inkubiert und die Änderung der Absorption durch die Wirkung der Proben photometrisch bestimmt. Als Ergebnis ist ein außerordentlich hohes antioxidatives Potential von P11599 festzustellen, das dem des relativ starken Reduktionsmittels Ascorbinsäure ähnelt (s. Abb. 8 und 9).

### Beispiel 11:

### Messung der cytotoxischen Wirkung und Einfluß auf Reduktionskapazität der Zellen

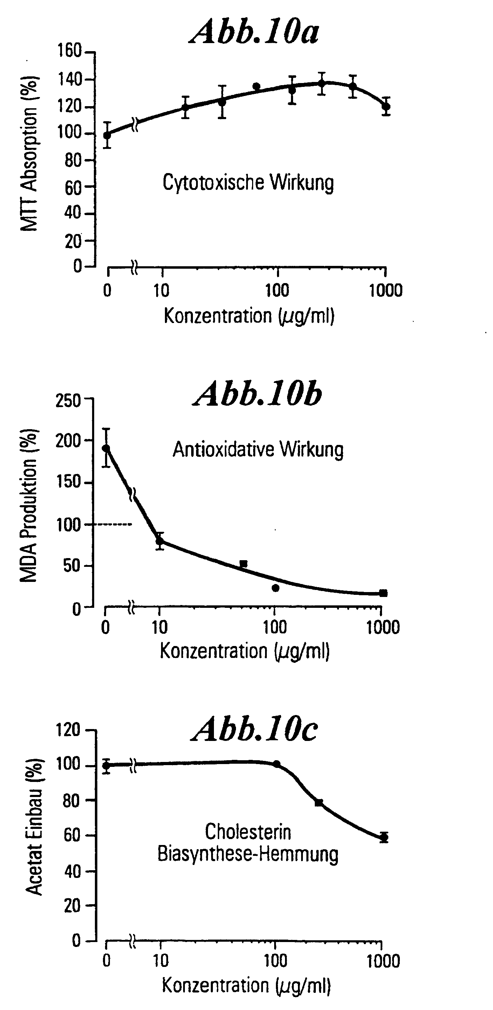

Gemäß Versuchsprotokoll von Gebhardt R. (1997) Toxicol. Appl. Pharmacol. 144, 279-286 wurde an kultivierten Rattenhepatozyten die cytotoxische Wirkung eines erfindungsgemäßen Extrakts P11599 gem. Beispiel 5 in verschiedenen Konzentrationen getestet. Dabei war - gemessen über die MIT-Absorption [%] - bis zu einer Konzentration von 1 mg/ml keine cytotoxische Wirkung auf Hepatozyten nachweisbar (Abb. 10). Als weiteres überraschendes Ergebnis zeigt diese Experiment durch den Anstieg der Kurve, daß unter der Wirkung des erfindungsgemäßen Extraktes sogar die reduktive Kapazität der Zellen zunimmt, was einer Erhöhung der Reduktionskapazität zur Erhaltung des Glutathion-Spiegels entspricht (Abb. 10).

### Beispiel 12:

### Messung der Verringerung der Smooth-Muscle-alpha-Aktin (SMA) Expression

Es wurden ruhende hepatische Sternzellen aus einer Rattenleber durch Perfusion der Rattenleber in situ mittels Kollagenase und Pronase gewonnen. Aus der resultierenden Zellsuspension wurden die hepatischen Sternzellen (HSC) über einen Dichtegradienten mit Nycodenz™ gereinigt. Anschließend wurden die isolierten HSC in Zellkultur genommen (ruhender, nicht aktivierter Phänotyp). Unter Standard-Kultivierungsbedingungen in Nährkulturmedium (DMEM, 18 % fötales Kälberserum, herkömmlich zu beziehen bei Sigma, Deisenhofen, Deutschland) findet eine spontane Aktivierung der HSC (aktivierter Phänotyp) statt. Zur weiteren Aktivierung wurden die HSC in Zellkultur passagiert. Diese Zellen wurden morphologisch und immun-histochemisch als myofibroblastische HSC charakterisiert.

Für die Durchführung der eigentlichen Testreihen wurden dem Nährkulturmedium der aktivierten HSC erfindungsgemäße Phyllanthusextrakte (25 % Ethanol LAT-Nr. 02700514, 50 % Ethanol LAT-Nr. 0271614, 75 % Ethanol LAT-Nr. 0272514) gelöst in DMSO in Konzentrationen von 20 µg/ml, 60 µg/ml und 200 µg/ml zugesetzt. Anschließend wurden die behandelten HSC für vier Tage in Nährkulturmedium inkubiert. Nach der Ernte der HSC wurde nach herkömmlichen Verfahren gesamtzelluläre RNA isoliert, deren Konzentration und Qualität spektrophotometrisch und mittels Agarose-Gelelektrophorese bestimmt. Der Nachweis der Expression der SMA mRNA erfolgte mittels dem im Stand der Technik bekannten Northemblot-Verfahren.

Bei allen getesteten Phyllanthusextrakten führte der Zusatz von 200 µg/ml zu einer deutlichen Inhibition des Wachstums der HSC in Zellkultur und einer wesentlich geringeren Menge an isolierter gesamtzellulärer RNA. Im Northemblot wurde eine ebenfalls deutliche Reduktion der Expression der SMA mRNA beobachtet, die sich bei Verwendung des 50 % Ethanolextraktes bereits bei einer Konzentration von 60 µg/ml zeigte.

### Referenzbeispiel 13:

### Messung der Verringerung der Expression der LPS-induzierten Stickstoffmonoxid-Synthase (NOS), des induzierten NOS Proteins (iNOS) und des Cyclooxygenase (COX-2) Proteins

Für die Messung der Stickstoffmonoxid (NO) Produktion, welche durch NOS erfolgt, wurden Makrophagen verwendet, die in RPMI Nährkulturmedium mit 10 % fötalem Kälberserum (herkömmlich zu beziehen bei Sigma, Deisenhofen, Deutschland) inkubiert wurden. Die Zellen wurden anschließend mit LPS (1 µg/ml) stimuliert und für 24 h mit oder zur Kontrolle ohne Phyllanthusextrakte inkubiert. Die NO Konzentration wurde mittels Griess Assay (Kiemer, A. und Vollmar, A. (1998), J. Biol. Chem. 273(22):13444-13451) bestimmt.

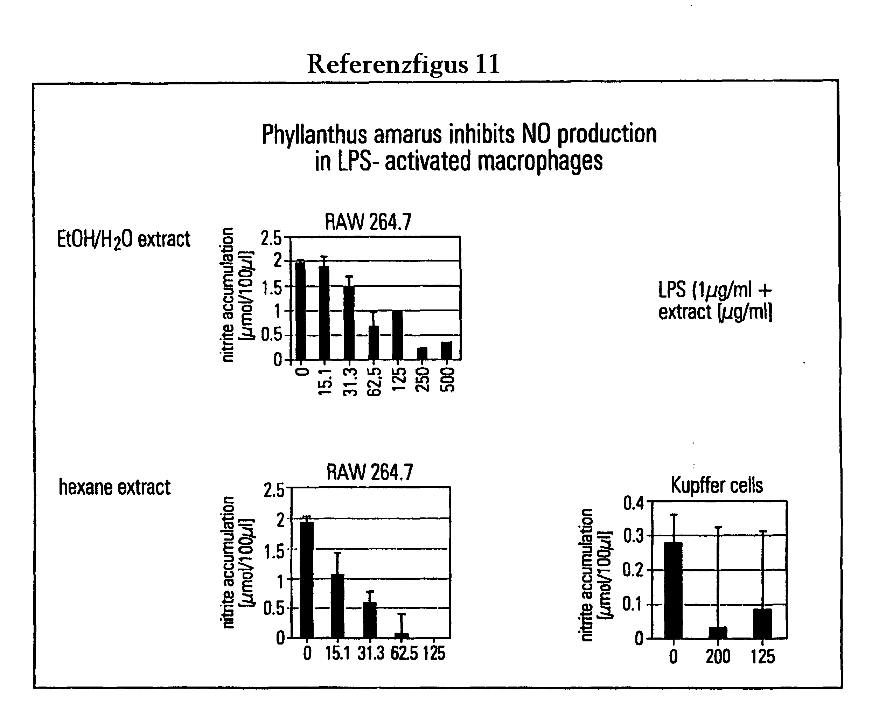

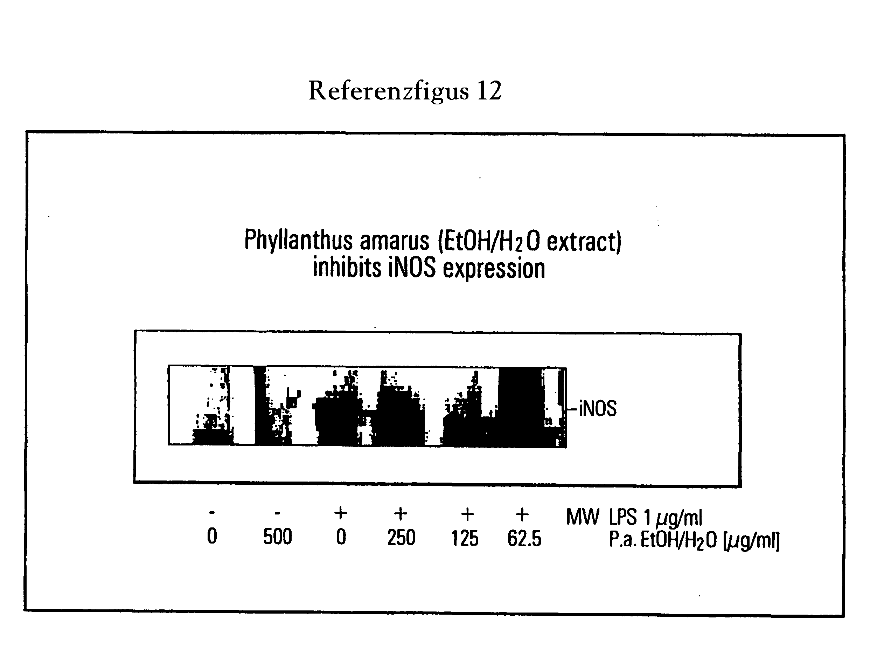

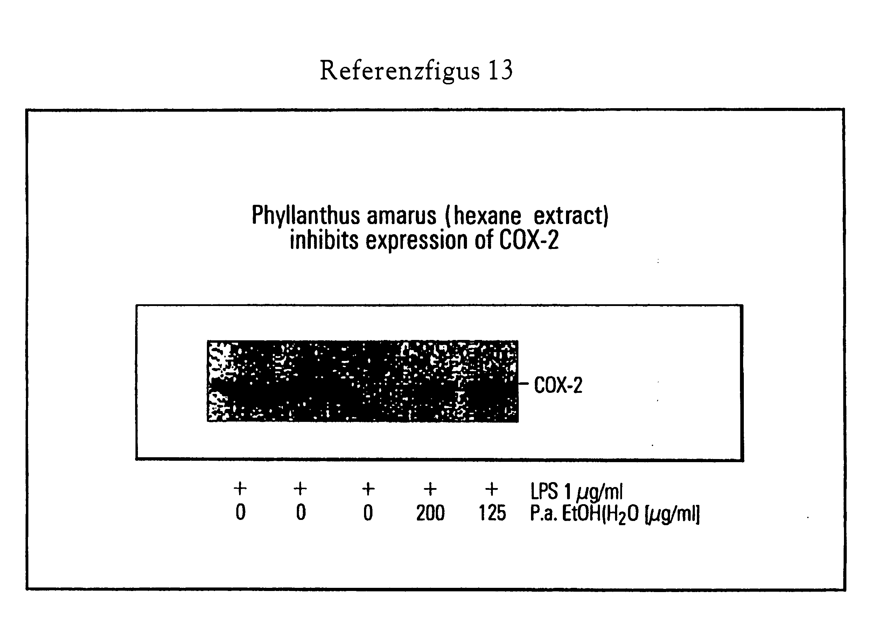

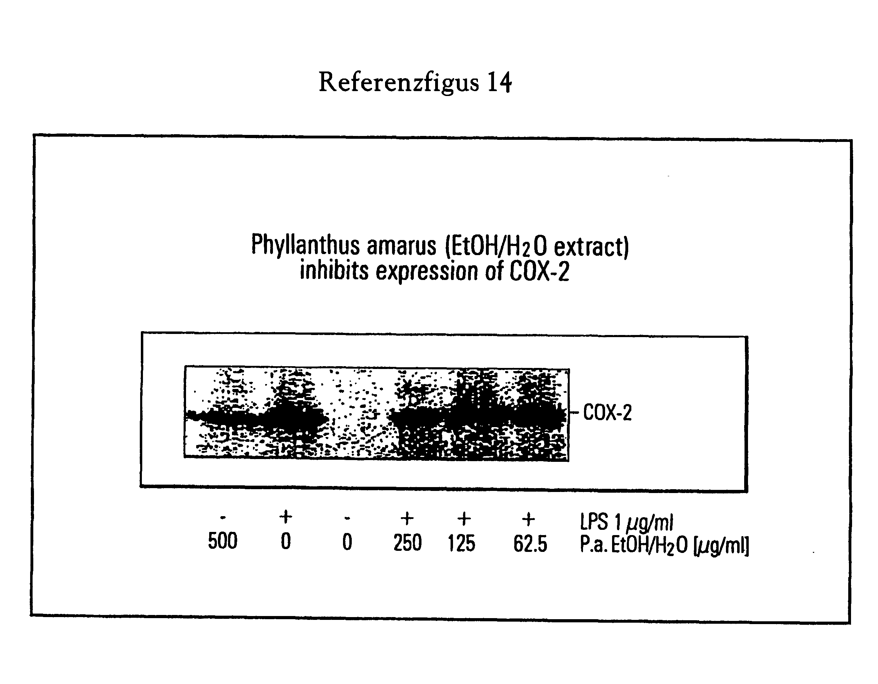

Bei den getesteten Phyllanthusextrakten reduzierten vor allem ein Hexanextrakt (LAT-Nr. 01600514) bereits in einer Konzentration von 12,5 µg/ml und ein Ethanol/Wasser Extrakt (LAT-Nr. 00690514) in einer Konzentration von 250 µg/ml wirksam die NO Produktion. Eine Reduktion der LPS-induzierten iNOS und COX-2 Proteinexpression wurde bei Konzentrationen von 125 µg/ml Hexanextrakt und Ethanol/Wasser Extrakt festgestellt (Abb. 11 - 14).

## Patentansprüche

1. Verwendung von Phyllanthus zur Herstellung eines Arzneimittels zur Behandlung oder Prävention von Bindegewebsvermehrungen.

2. Verwendung nach Anspruch 1, wobei die Bindegewebsvermehrungen fibrotische Veränderungen der Leber, der Lunge, der Niere, des Pankreas, des Darms, von endokrinen Organen, der Milz, des männlichen oder weiblichen Urogenitaltraktes oder der Gelenke oder Leberzirrhose umfassen.

3. Verwendung nach Anspruch 1 oder 2, wobei eine aus Phyllanthus isolierte Fraktion verwendet wird.

4. Verwendung gemäß einem der Ansprüche 1 bis 3, wobei eine oder mehrere aus Phyllanthus isolierte chemische Substanzen, verwendet werden.

5. Verwendung gemäß einem der Ansprüche 1 bis 4, wobei Phyllanthus ausgewählt wird aus einzelnen Mitgliedern der Gattung Phyllanthus aus der Gruppe Phyllanthus amarus, Phyllanthus niruri, Phyllanthus emblica, Phyllanthus urinaria, Phyllanthus myrtifolius Moon, Phyllanthus maderas pratensis und/oder Phyllanthus ussuriensis.

6. Verwendung gemäß einem der Ansprüche 1 bis 5, wobei Blätter, Rinde, Blüten, Samen, Früchte, Stengel, Äste, Stamm, Wurzel, Holz und/oder die Herba von Phyllanthus verwendet werden.

7. Verwendung gemäß einem der Ansprüche 1 bis 6, wobei Phyllanthus in zerkleinerter Form, in unveränderter Form, als Granlulat, Pulver, Präzipitat, Extrakt, getrockneter Extrakt und/oder Exsudat eingesetzt wird.

8. Verwendung gemäß Anspruch 7, wobei ein wässriger unpolarer, verzweigt- oder unverzweigt-kettiger Kohlenwasserstoff, oder Mischungen davon, vor allem mit n-Hexan, und/oder alkoholischer Extrakte, primärer Alkohole oder Mischungen davon verwendet wird.

9. Verwendung nach Anspruch 8, wobei der wässrige unpolare Kohlenwasserstoff C5-C10 verzweigt- oder unverzweigt-kettige Kohlenwasserstoffe umfaßt.

10. Verwendung nach Anspruch 8 oder 9, wobei der alkoholische Extrakt kurzkettige C1 bis C4 primäre Alkohole oder Mischungen davon umfaßt.

11. Verwendung nach Anspruch 10, wobei der primäre Alkohol Ethanol oder Methanol ist.

12. Verwendung gemäß einem der Ansprüche 1 bis 11, wobei die Anwendung in Form einer Infusionslösung, Injektionslösung, Tablette, eines Granulats einer Salbe, von Klysmen, einer Heilpackung und/oder eines Nahrungsergähzungsmittels erfolgt.

13. Verwendung gemäß einem der Ansprüche 1 bis 12, wobei die Anwendung oral, topisch und/oder parenteral erfolgt.

14. Verwendung gemäß einem der Ansprüche 1 bis 13, wobei zusätzlich zu Phyllanthus andere Wirkstoffe verwendet werden und gegebenenfalls geeignete Zusatz- und/oder Hilfsstoffe.

15. Verwendung gemäß Anspruch 14, wobei Ascorbinsäure und/oder Tocopherole verwendet werden.

## Claims

1. Use of Phyllanthus for producing a pharmaceutical composition for treating or preventing connective tissue proliferation.

2. Use according to claim 1, wherein the connective tissue proliferation comprises fibrotic changes in the liver, the lung, the kidney, the pancreas, the intestine, of endocrine organs, the spleen, the male or female urogenital tract or the joints or cirrhosis of the liver.

3. Use according to claim 1 or 2, wherein a fraction isolated from Phyllanthus is used.

4. Use according to any one of claims 1 to 3, wherein one or more chemical substances isolated from Phyllanthus are used.

5. Use according to any one of claims 1 to 4, wherein Phyllanthus is selected from individual members of the genus Phyllanthus from the group of Phyllanthus amarus, Phyllanthus niruri, Phyllanthus emblica, Phyllanthus urinaria, Phyllanthus myrtifolius Moon, Phyllanthus maderas pratensis and/or Phyllanthus ussuriensis.

6. Use according to any one of claims 1 to 5, wherein leaves, bark, flowers, seeds, fruits, stalks, branches, stem, root, wood and/or the herb of Phyllanthus are used.

7. Use according to any one of claims 1 to 6, wherein Phyllanthus is used in ground form, in unchanged form, as granulate, powder, precipitate, extract, dried extract and/or exudate.

8. Use according to claim 7, wherein an aqueous non-polar, branched or linear hydrocarbon, or mixtures thereof, mainly with n-hexane, and/or alcoholic extracts, primary alcohols or mixtures thereof are used.

9. Use according to claim 8, wherein the aqueous non-polar hydrocarbon comprises C5-C10 branched or linear hydrocarbons.

10. Use according to claim 8 or 9, wherein the alcoholic extract comprises short-chain C1 to C4 primary alcohols or mixtures thereof.

11. Use according to claim 10, wherein the primary alcohol is ethanol or methanol.

12. Use according to any one of claims 1 to 11, wherein the administration is carried out in form of an infusion solution, an injection solution, a tablet, a granulate, an ointment, clysma, a medicinal pack and/or a food supplement.

13. Use according to any one of claims 1 to 12, wherein the administration is carried out orally, topically and/or parenterally.

14. Use according to any one of claims 1 to 13, wherein other active agents and, optionally, suitable additives and/or adjuvants are used in addition to Phyllanthus.

15. Use according to claim 14, wherein ascorbic acid and/or tocopherols are used.

## Revendications

1. Utilisation de Phyllanthus pour la préparation d'un médicament pour le traitement ou la prévention de proliférations du tissu conjonctif.

2. Utilisation selon la revendication 1, dans laquelle les proliférations du tissu conjonctif comprennent des modifications fibrotiques du foie, du poumon, des reins, du pancréas, de l'intestin, des organes endocriniens, de la rate, du tractus urogénital masculin ou féminin ou des articulations ou une cirrhose du foie.

3. Utilisation selon la revendication 1 ou 2, dans laquelle on utilise une fraction isolée de Phyllanthus.

4. Utilisation selon l'une des revendications 1 à 3, dans laquelle on utilise une ou plusieurs substances chimiques isolées de Phyllanthus.

5. Utilisation selon l'une des revendications 1 à 4, dans laquelle Phyllanthus est choisi parmi les membres individuels du genre Phyllanthus du groupe Phyllanthus amarus, Phyllanthus niruri, Phyllanthus emblica, Phyllanthus urinaria, Phyllanthus myrtifolius moon, Phyllanthus maderas pratensis et/ou Phyllanthus ussuriensis.

6. Utilisation selon l'une quelconque des revendications 1 à 5, dans laquelle on utilise les feuilles, écorces, fleurs, semences, fruits, tiges, branches, troncs, racines, bois et/ou l'herbe de Phyllanthus.

7. Utilisation selon l'une des revendications 1 à 6, dans laquelle Phyllanthus est mis en oeuvre sous forme réduite en petits morceaux, sous forme non modifiée, sous forme de granulat, poudre, précipité, extrait, extrait sec et/ou exsudat.

8. Utilisation selon la revendication 7, dans laquelle on utilise un hydrocarbure apolaire aqueux, à chaîne ramifiée ou linéaire, ou des mélanges de celui-ci, avec en particulier du n-hexane, et/ou des extraits alcooliques, des alcools primaires ou des mélanges de ceux-ci.

9. Utilisation selon la revendication 8, dans laquelle l'hydrocarbure apolaire aqueux comprend des hydrocarbures en C5-C10 à chaîne ramifiée ou linéaire.

10. Utilisation selon la revendication 8 ou 9, dans laquelle l'extrait alcoolique comprend des alcools primaires à chaîne courte en C1 jusqu'à C4 ou des mélanges de ceux-ci.

11. Utilisation selon la revendication 10, dans laquelle l'alcool primaire est de l'éthanol ou du méthanol.

12. Utilisation selon l'une des revendications 1 à 11, dans laquelle l'application est réalisée sous forme d'une solution pour perfusion, solution pour injection, comprimé, un granulat, un onguent, lavements, un pansement et/ou un complément alimentaire.

13. Utilisation selon l'une des revendications 1 à 12, dans laquelle l'application est faite sous forme orale, topique et/ou parentérale.

14. Utilisation selon l'une quelconque des revendications 1 à 13, dans laquelle on utilise outre Phyllanthus d'autres agents actifs et éventuellement des additifs et/ou auxiliaires appropriés.

15. Utilisation selon la revendication 14, dans laquelle on utilise de l'acide ascorbique et/ou des tocophérols.
